# EUROPÄISCHE PATENTSCHRIFT

(11) **EP 1 485 337 B1**
(45) Veröffentlichungstag und Bekanntmachung des Hinweises auf die Patenterteilung: **02.12.2015**
(21) Anmeldenummer: 03743835.5
(22) Anmeldetag: 04.03.2003
(51) Int. Cl.: C07C 5/333, C07C 45/35, B01J 8/02, B01J 19/26, B01J 23/00, B01J 27/199, C07C 45/33

(54) **VERFAHREN DER KONTINUIERLICHEN HETEROGEN KATALYSIERTEN PARTIELLEN DEHYDRIERUNG**
PARTIAL DEHYDROGENATION METHOD USING CONTINUOUS HETEROGENEOUS CATALYSIS
PROCEDE DE DESHYDROGENATION PARTIELLE CONTINUE PAR CATALYSE HETEROGENE

(30) Priorität: 13.03.2002 DE 10211275
(43) Veröffentlichungstag der Anmeldung: 15.12.2004
(73) Patentinhaber: BASF SE, 67056 Ludwigshafen (DE)
(72) Erfinder: MACHHAMMER, Otto, 68163 Mannheim (DE); SCHINDLER, Götz-Peter, 68219 Mannheim (DE); HARTH, Klaus, 67317 Altleiningen (DE); ZEHNER, Peter, 67071 Ludwigshafen (DE)
(86) Internationale Anmeldenummer: PCT/EP2003/002192
(87) Internationale Veröffentlichungsnummer: WO 2003/076370

(56) Entgegenhaltungen:
- WO-A-01/96008
- WO-A-97/36849
- US-A- 3 542 889

## Beschreibung

Vorliegende Erfindung betrifft ein Verfahren der kontinuierlichen heterogen katalysierten partiellen Dehydrierung wenigstens eines zu dehydrierenden Kohlenwasserstoffs in der Gasphase, bei dem
- einer Reaktionszone ein den wenigstens einen zu dehydrierenden Kohlenwasserstoff enthaltendes Reaktionsgas kontinuierlich zugeführt wird,
- das Reaktionsgas in der Reaktionszone über wenigstens ein Katalysatorfestbett geführt wird, an welchem durch katalytische Dehydrierung molekularer Wasserstoff und partiell wenigstens ein dehydrierter Kohlenwasserstoff gebildet werden,
- dem Reaktionsgas vor und/oder nach Eintritt in die Reaktionszone wenigstens ein molekularen Sauerstoff enthaltendes Gas zugesetzt wird,
- der molekulare Sauerstoff in der Reaktionszone im Reaktionsgas enthaltenen molekularen Wasserstoff teilweise zu Wasserdampf oxidiert und
- der Reaktionszone ein Produktgas entnommen wird, das molekularen Wasserstoff, Wasserdampf, den wenigstens einen dehydrierten Kohlenwasserstoff und den wenigstens einen zu dehydrierenden Kohlenwasserstoff enthält.

Außerdem betrifft vorliegende Erfindung eine Vorrichtung zur Durchführung des erfindungsgemäßen Verfahrens.

Der in dieser Anmeldung verwendete Begriff "dehydrierte Kohlenwasserstoffe" soll acyclische und cyclische aliphatische Kohlenwasserstoffe mit einer oder mehreren C,C-Doppelbindungen im Molekül umfassen. Beispiele für solche aliphatische Kohlenwasserstoffe sind Propen, iso-Buten, 1-Buten, 2-Buten und Butadien. D.h., zu den dehydrierten Kohlenwasserstoffen gehören insbesondere die einfach ungesättigten linearen (n-Alkene) oder verzweigten aliphatischen Kohlenwasserstoffe (z.B. iso-Alkene), sowie die Cycloalkene. Ferner sollen die dehydrierten Kohlenwasserstoffe auch die Alkapolyene (z.B. Diene und Triene) umfassen, die mehr als eine Kohlenstoff-Kohlenstoff-Doppelbindung im Molekül enthalten. Er soll aber auch Kohlenwasserstoffverbindungen umfassen, die ausgehend von Alkylaromaten wie Ethylbenzol oder Isopropylbenzol durch Dehydrierung des Alkylsubstituenten erhältlich sind. Das sind z.B. Verbindungen wie Styrol oder α-Methylstyrol.

Ganz generell bilden dehydrierte Kohlenwasserstoffe wertvolle Ausgangsverbindungen zur Synthese von z.B. funktionalisierten, radikalisch polymerisierbaren, Verbindungen (z.B. Acrylsäure aus Propen oder Methacrylsäure aus Isobuten) und deren Polymerisationsprodukten. Sie eignen sich aber auch zur Herstellung von Verbindungen wie Methyl-tert.-butyl-ether (Folgeprodukt von iso-Buten, das z.B. als Kraftstoffadditiv zur Erhöhung der Oktanzahl geeignet ist). Dehydrierte Kohlenwasserstoffe können aber auch als solche selbst zur Polymerisation verwendet werden.

Es ist allgemein bekannt, dehydrierte Kohlenwasserstoffe durch kontinuierliche heterogen katalysierte partielle Dehydrierung von zu dehydrierenden Kohlenwasserstoffen in der Gasphase herzustellen (vgl. z.B. DE-A 10028582, DE-A 10047642, DE-A 19937107 und in diesen Schriften zitierte Literatur).

Als zu dehydrierende Kohlenwasserstoffe kommen dabei insbesondere die acyclischen und cyclischen Alkane, aber auch Olefine (deren C,C-Doppelbindungszahl erhöht werden soll) in Betracht (als Beispiel sei die heterogen katalysierte partielle Dehydrierung von n-Butenen zu Butadien erwähnt).

D.h., der Begriff "zu dehydrierende Kohlenwasserstoffe" umfaßt in dieser Patentanmeldung insbesondere C₂- bis C₁₆-Alkane wie z.B. Ethan, Propan, n-Butan, iso-Butan, n-Pentan, iso-Pentan, n-Hexan, n-Heptan, n-Octan, n-Nonan, n-Decan, n-Undecan, n-Dodecan, n-Tridecan, n-Tetradecan, n-Pentadecan und n-Hexadecan.

Insbesondere aber gelten alle in dieser Schrift getroffenen Aussagen für C₂- bis C₈-Alkane als zu dehydrierende Kohlenwasserstoffe und ganz besonders für C₂- bis C₄-Kohlenwasserstoffe als zu dehydrierende Kohlenwasserstoffe.

D.h., zu dehydrierende Kohlenwasserstoffe sind in dieser Schrift vor allem Ethan, Propan, n-Butan und iso-Butan, aber auch 1-Buten und 2-Buten.

Heterogen katalysierte partielle Dehydrierungen von zu dehydrierenden Kohlenwasserstoffen benötigen vergleichsweise hohe Reaktionstemperaturen. Der dabei erzielbare Umsatz ist normalerweise durch das thermodynamische Gleichgewicht begrenzt. Typische Reaktionstemperaturen betragen 300 bis 700°C. Pro Molekül dehydriertem Kohlenwasserstoff wird dabei in der Regel wenigstens ein Molekül Wasserstoff erzeugt.

Charakteristisch für heterogen katalysierte partielle Dehydrierungen von zu dehydrierenden Kohlenwasserstoffen ist ferner, daß sie endotherm verlaufen. Die für die Einstellung eines gewünschten Umsatzes benötigte Dehydrierwärme muss daher dem Reaktionsgas entweder vorab und/oder im Verlauf der katalytischen Dehydrierung zugeführt werden.

Bei der Mehrzahl der bekannten Verfahren der heterogen katalysierten partiellen Dehydrierung von zu dehydrierenden Kohlenwasserstoffen wird die Dehydrierwärme außerhalb des Reaktors erzeugt und dem Reaktionsgas von außen zugeführt. Dies erfordert jedoch aufwendige Reaktor- und Verfahrenskonzepte und führt insbesondere bei hohen Umsätzen zu steilen Temperaturgradienten im Reaktor, mit dem Regelnachteil einer erhöhten Nebenproduktbildung.

Alternativ kann die Dehydrierwärme auch unmittelbar im Reaktionsgas selbst dadurch erzeugt werden, daß man durch Zusatz von molekularem Sauerstoff entweder bei der Dehydrierung gebildeten oder zusätzlich zugeführten Wasserstoff exotherm zu Wasserdampf verbrennt. Dazu wird dem Reaktionsgas vor und/oder nach Eintritt in die den Dehydrierkatalysator enthaltende Reaktionszone ein sauerstoffhaltiges Gas und gegebenenfalls Wasserstoff zugesetzt. Entweder der Dehydrierkatalysator selbst (dies gilt für die meisten Dehydrierkatalysatoren) und/oder zusätzlich eingebrachte Oxidationskatalysatoren erleichtern im Regelfall die erwünschte Wasserstoffoxidation (vgl. DE-A 10028582). Solchermaßen mittels Wasserstoffverbrennung freigesetzte Reaktionswärme ermöglicht in günstigen Fällen den völligen Verzicht auf eine indirekte Reaktorheizung und damit vergleichsweise einfache Verfahrenskonzepte sowie auch bei hohen Umsätzen beschränkte Temperaturgradienten im Reaktor.

Nachteilig an den Verfahren der direkten Wärmeerzeugung mittels Wasserstoffverbrennung im Reaktionsgas ist jedoch, daß sie entweder in signifikantem Umfang des Zusatzes von externem molekularem Wasserstoff bedürfen, der vergleichsweise teuer ist, oder daß die Selektivität der Bildung des dehydrierten Kohlenwasserstoffs nicht voll zu befriedigen vermag, da der zugesetzte molekulare Sauerstoff bei alleiniger Zugriffsmöglichkeit auf während der heterogen katalysierten partiellen Dehydrierung gebildeten Wasserstoff regelmäßig nicht nur diesen molekularen Wasserstoff sondern häufig auch eine Teilmenge an zu dehydrierendem und/oder an bereits dehydriertem Kohlenwasserstoff verbrennt, was die Zielproduktselektivität in unerwünschter Weise mindert.

Die Aufgabe der vorliegenden Erfindung bestand daher darin, ein wie eingangs definiertes Verfahren der kontinuierlichen heterogen katalysierten partiellen Dehydrierung wenigstens eines zu dehydrierenden Kohlenwasserstoffs in der Gasphase zur Verfügung zu stellen, das die vorgenannten Nachteile entweder nicht mehr und/oder nur noch in geminderter Form aufweist.

Demgemäß wurde ein Verfahren der kontinuierlichen heterogen katalysierten partiellen Dehydrierung wenigstens eines zu dehydrierenden Kohlenwasserstoffs in der Gasphase gefunden, bei dem
- einer Reaktionszone ein den wenigstens einen zu dehydrierenden Kohlenwasserstoff enthaltendes Reaktionsgas kontinuierlich zugeführt wird,
- das Reaktionsgas in der Reaktionszone über wenigstens ein Katalysatorfestbett geführt wird, an welchem durch katalytische Dehydrierung molekularer Wasserstoff und partiell wenigstens ein dehydrierter Kohlenwasserstoff gebildet werden,
- dem Reaktionsgas vor und/oder nach Eintritt in die Reaktionszone wenigstens ein molekularen Sauerstoff enthaltendes Gas zugesetzt wird,
- der molekulare Sauerstoff in der Reaktionszone im Reaktionsgas enthaltenen molekularen Wasserstoff teilweise zu Wasserdampf oxidiert und
- der Reaktionszone ein Produktgas entnommen wird, das molekularen Wasserstoff, Wasserdampf, den wenigstens einen dehydrierten Kohlenwasserstoff und den wenigstens einen zu dehydrierenden Kohlenwasserstoff enthält,
dadurch gekennzeichnet, daß das der Reaktionszone entnommene Produktgas in zwei Teilmengen identischer Zusammensetzung aufgeteilt und eine der beiden Teilmengen als Kreisgas in die Reaktionszone zurückgeführt wird.

Das erfindungsgemäße Verfahren eignet sich insbesondere für die heterogen katalysierte partielle Dehydrierung von Propan zu Propen, von Butan zu Buten und/oder Butadien sowie von Butenen zu Butadien.

Es unterscheidet sich von der katalytischen Oxidehydrierung von Kohlenwasserstoffen insbesondere dadurch, daß bei der katalytischen Oxidehydrierung kein Wasserstoff gebildet wird. Vielmehr wird dort der dem zu dehydrierenden Kohlenwasserstoff entrissene Wasserstoff unmittelbar als Wasser (H₂O) entrissen. Dazu bedarf es in der Regel anderer Reaktionsbedingungen und anderer Katalysatoren.

Prinzipiell können für das erfindungsgemäße Verfahren alle Dehydrierkatalysatoren eingesetzt werden, die im Stand der Technik für heterogen katalysierte partielle Dehydrierungen von zu dehydrierenden Kohlenwasserstoffen in der Gasphase unter Bildung von molekularem Wasserstoff empfohlen werden.

Sie lassen sich grob in zwei Gruppen unterteilen. Nämlich in solchen, die oxidischer Natur sind (z.B. Chromoxid und/oder Aluminiumoxid) und in solche, die aus wenigstens einem auf einem, in der Regel oxidischen, Träger abgeschiedenen, in der Regel vergleichsweise edlen, Metall (z.B. Platin) bestehen. Lewis-acide Dehydrierkatalysatoren, die im wesentlichen keine Brönsted-Acidität aufweisen, gemäß der DE-A 10047642 sind erfindungsgemäß bevorzugt.

Geeignet sind somit insbesondere alle Dehydrierkatalysatoren die in der DE-A 10060099 (das Ausführungsbeispiel), der WO 99/46039, der US-A 4788371, der EP-A 705136, der WO 99/29420, der US-A 5220091, der US-A 5,430,220, der US-A 5877369, der EP-A 117146, der DE-A 19937106, der DE-A 19937105, der DE-A 10047642 sowie der DE-A 19937107 empfohlen werden.

Im besonderen können für alle in dieser Schrift angesprochenen Varianten des erfindungsgemäßen Verfahrens sowohl der Katalysator gemäß Beispiel 1, als auch gemäß Beispiel 2, als auch gemäß Beispiel 3, als auch gemäß Beispiel 4 der DE-A 19937107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumoxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, daß die Summe der Gewichtsprozente 100 Gew.-% ergibt.

Die Durchführung des erfindungsgemäßen Verfahrens an im festen Zustand befindlichen selektiv wirkenden Katalysatoren ist dadurch bedingt, daß die Dehydrierung (Spaltung von C-H) gegenüber der Crackung (Spaltung von C-C) kinetisch benachteiligt ist. Infolge der selektiv wirkenden Katalysatoren, die üblicherweise so beschaffen sind, daß sie unter Ausschluß von Sauerstoff bei Temperaturen von 300 bis 700°C (z.B. bei 600°C) eine signifikante Dehydrierung entfalten (im Fall von Propan als zu dehydrierendem Kohlenwasserstoff beträgt die Propylenausbeute bei Propan-Belastungen der Katalysatoren von z.B. 1000 h⁻¹ wenigstens 30 mol-% im einmaligen Durchgang (bezogen auf eingesetztes Propan)), entstehen Nebenprodukte wie Methan, Ethylen und Ethan nur in untergeordneten Mengen.

Das im Rahmen des erfindungsgemäßen Verfahrens erforderliche wenigstens eine Katalysatorfestbett kann den Dehydrierungskatalysator zu unterschiedlichen Geometrien geformt enthalten. Für das erfindungsgemäße Verfahren geeignete Geometrien sind z.B. Formen wie Splitt, Tabletten, Monolithe, Kugeln oder Extrudate (Stränge, Wagenräder, Sterne, Ringe). Die Länge im Fall von Extrudaten beträgt dabei zweckmäßig 2 bis 15 mm, häufig 2 bis 10 mm, vielfach 6 bis 10 mm und der Durchmesser des Extrudatquerschnitts beträgt zweckmäßig 1 bis 5 mm, häufig 1 bis 3 mm. Die Wanddicke beträgt im Fall von Ringen vorteilhaft 0,3 bis 2,5 mm, ihre Länge 2 bis 15 mm, häufig 5 bis 15 mm und der Durchmesser ihres Querschnitts 3 bis 10 mm. Ein geeignetes Formgebungsverfahren offenbart z.B. die DE-A 10047642 sowie die DE-A 19937107. Es fußt darauf, daß oxidische Trägermaterialien mit konzentrierter Mineralsäure (z.B. konzentrierte Salpetersäure) versetzt sich vergleichsweise gut kneten und mittels eines Extruders oder einer Strangpresse in einen entsprechenden Formkörper überführen lassen.

Die Formkörper werden dann getrocknet, calciniert und anschlie-ßend in bestimmter Reihenfolge mit Salzlösungen übergossen. Abschließend wird wieder getrocknet und calciniert.

In der weiteren Beschreibung wird vielfach spezifisch auf den erfindungsgemäß bevorzugten Fall der Propan-Dehydrierung eingegangen. Die dabei gemachten Ausführungen gelten jedoch in analoger Weise auch für andere dehydrierbare Kohlenwasserstoffe.

Die für das erfindungsgemäße Verfahren relevante Reaktionszone kann grundsätzlich in allen aus dem Stand der Technik für heterogen katalysierte partielle Dehydrierungen von Kohlenwasserstoffen in der Gasphase an Festbettkatalysatoren bekannten Reaktortypen realisiert werden. Typische Reaktionstemperaturen liegen bei 200 bis 800°C, bzw. 400 bis 650°C. Der Arbeitsdruck liegt typisch im Bereich von 0,5 bis 10 bar. Typische Katalysatorbelastungen mit Reaktionsgas betragen 300 bis 16000 h⁻¹.

Eine ausführliche Beschreibung geeigneter Reaktortypen enthält z.B. "Catalytica® Studies Division, Oxidative Dehydrogenation and Alternative Dehydrogenation Processes, Study Number 4192 OD, 1993, 430 Ferguson Drive, Mountain View, California, 94043-5272 U.S.A.".

Eine geeignete Reaktorform ist der Festbettrohr- oder Rohrbündelreaktor. Bei diesen befindet sich der Dehydrierungskatalysator (und gegebenenfalls spezifischer Wasserstoffoxidationskatalysator, wie er z.B. in den Schriften US-A 4788371, US-A 4886928, US-A 5430209, US-A 55530171, US-A 5527979, US-A 5563314 und EP-A 832056 offenbart wird) als Festbett im Reaktionsrohr oder in einem Bündel von Reaktionsrohren. Die Reaktionsrohre werden üblicherweise dadurch indirekt beheizt, daß im die Reaktionsrohre umgebenden Raum ein Gas, z.B. ein Kohlenwasserstoff wie Methan, verbrannt wird. Günstig ist es, diese indirekte Form der Aufheizung lediglich auf den ersten etwa 20 bis 30 % der Festbettschüttung anzuwenden und die verbleibende Schüttungslänge durch die im Rahmen der Verbrennung freigesetzte Strahlungswärme auf die erforderliche Reaktionstemperatur aufzuwärmen. Das indirekte Aufheizen des Reaktionsgases wird erfindungsgemäß in vorteilhafter Weise mit direktem Aufheizen durch Verbrennung mit molekularem Sauerstoff im Reaktionsgas gekoppelt.

Durch diese Kopplung der erfindungsgemäßen direkten Wärmeeinbringung mit der indirekten Wärmeeinbringung ist in vergleichsweise einfacher Form eine im wesentlichen isotherme Reaktionsführung erreichbar.

Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 700°C, vorzugsweise im Bereich von 400 bis 700°C. Der Arbeitsdruck liegt üblicherweise im Bereich von 0,5 bis 8 bar, häufig bei 1 bis 2 bar oder auch bei 3 bis 8 bar. In der Regel verlässt das Produktgas das Reaktionsrohr und damit auch die Reaktionszone mit einer anderen (höheren oder tieferen) Temperatur als der Eingangstemperatur (vgl. auch US-A 4902849, US-A 4996387 und US-A 5389342). Typische Katalysatorbelastungen mit zu dehydrierendem Kohlenwasserstoff, z.B. Propan, liegen bei 500 bis 2000 h⁻¹ (= Nl/l Katalysator · h) .

Selbstverständlich kann das erfindungsgemäße Verfahren auch in einem Wanderbett-Reaktor durchgeführt werden (im Unterschied zum Wirbelbett soll in dieser Schrift das Wanderbett auch als Festbett betrachtet werden). Beispielsweise kann das Dehydrierkatalysator-Wanderbett in einem Radialstromreaktor untergebracht sein. In diesem bewegt sich der Katalysator langsam von oben nach unten, während das Reaktionsgas radial fließt. Es ist dabei zweckmäßig, mehrere Wanderbettreaktoren hintereinandergeschaltet zu betreiben. Dabei kann z.B. vor jedem Reaktor dem Reaktionsgas ein molekularen Sauerstoff enthaltendes Gas zugesetzt werden, dessen Verbrennung im jeweiligen Reaktor auf die Reaktionstemperatur aufheizt.

Je nachdem, welches einen der hintereinandergeschalteten Reaktoren verlassende Produktgas im erfindungsgemäßen Sinne in zwei Teilmengen aufgeteilt und je nachdem in welchen Reaktor die eine Teilmenge rückgeführt wird, kann die erfindungsgemäß relevante Reaktionszone die Gesamtmenge der hintereinandergeschalteten Reaktoren oder nur eine Teilmenge (z.B. jeden Reaktor für sich) umfassen. Sind beispielsweise vier Reaktoren hintereinandergeschaltet und wird nur das den vierten Reaktor verlassende Produktgas erfindungsgemäß in zwei Teilmengen aufgeteilt und eine der beiden Teilmengen in den ersten der vorgeschalteten Reaktoren rückgeführt, umfaßt die erfindungsgemäße Reaktionszone alle vier Reaktoren. Wird dagegen nur das den ersten der hintereinandergeschalteten Reaktoren verlassende Produktgas erfindungsgemäß in zwei Teilmengen aufgeteilt und eine der beiden Teilmengen in den ersten Reaktor rückgeführt, umfaßt die erfindungsgemäße Reaktionszone lediglich den ersten Reaktor.

Für Wanderbett-Reaktoren erfindungsgemäß eingesetzte Dehydrierkatalysatoren weisen in zweckmäßiger Weise Kugelform auf. Der Arbeitsdruck liegt in typischer Weise bei 2 bis 5 bar. Die Reaktionstemperaturen liegen in typischer Wesie bei 550 bis 660°C. Die Katalysatorbeschickung kann hier, wie auch im Fall aller anderen in dieser Schrift als für das erfindungsgemäße Verfahren als geeignet beschriebenen Reaktoren, mit einem Gemisch aus Dehydrier- und H₂-Oxidationskatalysatoren beschickt werden, wie es z.B. die EP-A 832056 empfiehlt.

Gemäß einer anderen Ausführungsform des erfindungsgemäßen Verfahrens wird die erfindungsgemäße heterogen katalysierte Dehydrierung in einem Hordenreaktor realisiert. Dieser enthält ein oder mehrere räumlich aufeinanderfolgende Katalysatorbetten. Die Anzahl der Katalysatorbetten kann 1 bis 20, zweckmäßigerweise 2 bis 8, insbesondere 4 bis 6 betragen. Die Katalysatorbetten werden vorzugsweise radial oder axial vom Reaktionsgas durchströmt.

Im einfachsten Fall sind die Katalysatorfestbetten in einem Schachtofenreaktor axial oder in den Ringspalten von zentrisch ineinander gestellten zylindrischen Gitterrosten angeordnet. Ein Schachtofenreaktor entspricht einer Horde. Die Durchführung des erfindungsgemäßen Verfahrens in einem einzelnen Schachtofenreaktor ist möglich.

Würde beim erfindungsgemäßen Verfahren dem Reaktionsgas vor und/ oder nach Eintritt in die Reaktionszone kein molekularen Sauerstoff enthaltendes Gas zugesetzt, würde das Reaktionsgas im Hordenreaktor auf seinem Weg von einem Katalysatorbett zum nächsten Katalysatorbett zweckmäßig einer Zwischenerhitzung unterworfen, z.B. durch Überleiten über mit heißen Gasen erhitzte Wärmeaustauscherrippen oder durch Durchleiten durch mit heißen Brenngasen beheizte Rohre.

Im Rahmen des erfindungsgemäßen Verfahrens wird vorstehend geschilderte Zwischenerhitzung wenigstens teilweise auf direktem Weg durchgeführt. Dazu wird dem Reaktionsgas entweder bereits vor Durchströmen des ersten Katalysatorbetts und/oder zwischen den nachfolgenden Katalysatorbetten in begrenztem Umfang ein molekularen Sauerstoff enthaltendes Gas zugesetzt.

An wenigsten einem, gegebenenfalls an allen, Katalysatorbetten wird so in begrenztem Umfang dem Reaktionsgas vorab zugesetzter und/oder im Verlauf der Dehydrierung bebildeter molekularer Wasserstoff verbrannt. Die dabei freigesetzte Reaktionswärme ermöglicht so eine im wesentlichen autotherme Betriebsweise der erfindungsgemäßen heterogen katalysierten Kohlenwasserstoffdehydrierung.

In einer Ausführungsform der Erfindung erfolgt eine Zwischeneinspeisung von sauerstoffhaltigem Gas gegebenenfalls vor jeder Horde des Hordenreaktors. In einer weiteren Ausführungsform des erfindungsgemäßen Verfahrens erfolgt die Einspeisung von sauerstoffhaltigem Gas vor jeder Horde außer der ersten Horde. In einer weiteren Ausführungsform des erfindungsgemäßen Verfahrens ist hinter jeder Sauerstoffeinspeisungsstelle eine Schüttung aus spezifischem, für Zwecke von H₂-Oxidationen geeignetem, Oxidationskatalysator vorhanden, gefolgt von einer Schüttung aus Dehydrierkatalysator. Bei Bedarf kann gegebenenfalls zusätzlich vor jeder Horde externer molekularer Wasserstoff zugeführt werden.

Erfindungswesentlich ist jedoch, daß es beim erfindungsgemäßen Verfahren keiner Einspeisung von externem molekularen Wasserstoff (damit ist molekularer Wasserstoff gemeint, der weder Bestandteil des in die Reaktionszone rückgeführten Kreisgases ist noch in der Reaktionszone selbst gebildet wird) in notwendiger Weise bedarf.

Die Dehydriertemperatur im Hordenreaktor beträgt im allgemeinen 400 bis 800°C, der Druck im allgemeinen 0,2 bis 10 bar, bevorzugt 0,5 bis 4 bar und besonders bevorzugt 1 bis 2 bar. Die Gesamtgasbelastung (GHSV) liegt in der Regel bei 500 bis 2000 h⁻¹, bei Hochlastfahrweise auch bis zu 16000 h⁻¹, regelmäßig 4000 bis 16000 h⁻¹.

Im erfindungsgemäßen Verfahren zu dehydrierende Kohlenwasserstoffe müssen keine Reinstoffe sein. Vielmehr kann der eingesetzte zu dehydrierende rohe Kohlenwasserstoff noch andere dehydrierbare Gase enthalten. Im Fall des Propans können dies Methan, Ethan, Ethylen, Propen, Butane, Butene, Propen, Acetylen, H₂S oder Pentane sein.

Das im Rahmen des erfindungsgemäßen Verfahrens dem Reaktionsgas vor und/oder nach Eintritt in die Reaktionszone zuzusetzende molekularen Sauerstoff enthaltende Gas kann entweder reiner molekularer Sauerstoff oder ein Gemisch mit Inertgasen wie CO₂, N₂ oder Edelgasen sein. Zweckmäßigerweise wird als molekularer Sauerstoff enthaltendes Gas normalerweise Luft verwendet.

In entsprechender Weise kann im Rahmen des erfindungsgemäßen Verfahrens der Reaktionszone gegebenenfalls extern zugesetzter molekularer Wasserstoff in reiner oder in mit Inertgas verdünnter Form zugesetzt werden.

Da die erfindungsgemäße heterogen katalysierte partielle Dehydrierung wenigstens eines zu dehydrierenden Kohlenwasserstoffs unter Volumenzunahme verläuft, kann der Umsatz durch Erniedrigung des Partialdrucks der Reaktanten gesteigert werden. Dies läßt sich in einfacher Weise z.B. durch Dehydrierung bei vermindertem Druck und/oder durch Zumischen eines Inertgases erreichen, weshalb die vorstehend genannten Inertgase im Rahmen des erfindungsgemäßen Verfahrens durchaus erwünscht sind:

Wie bereits erwähnt, kommen als solche für das erfindungsgemäße Verfahren geeignete Inertgase z.B. Stickstoff, Wasserdampf, Kohlendioxid und Edelgase wie He, Ne oder Ar in Betracht. Bevorzugte inerte Verdünnungsmittel sind solche, die sich unter den Reaktionsbedingungen des erfindungsgemäßen Verfahrens zu weniger als 5 mol-%, besonders bevorzugt zu weniger als 3 mol-% und noch besser zu weniger als 1 mol-% chemisch verändern). Alle genannten Verdünnungsmittel können beim erfindungsgemäßen Verfahren entweder für sich oder in Form unterschiedlichster Gemische mitverwendet werden. Als Verdünnungsgase können aber auch solche Gase verwendet werden, die unter den gegebenen Randbedingungen mit molekularem Sauerstoff schneller exotherm reagieren als der wenigstens eine dehydrierte und/oder zu dehydrierende Kohlenwasserstoff.

Prinzipiell kann das erfindungsgemäße Verfahren aber auch ohne Beisein von inerten, verdünnend wirkenden Gasen durchgeführt werden. D.h., erfindungsgemäße Verfahren sind auch solche, bei denen das Reaktionsgas, das der Reaktionszone zugeführt wird, ausschließlich aus dem wenigstens einen zu dehydrierenden Kohlenwasserstoff besteht. Das erfindungsgemäß erforderliche, molekularen Sauerstoff enthaltende, Gas wird in diesen Fällen erst längs des Reaktionspfades der Reaktionszone zugeführt. Es kann erfindungsgemäß jedoch auch bereits vorab des Eintritts des Reaktionsgases in die Reaktionszone selbigem zugesetzt werden. In diesen Fällen kann das Reaktionsgas somit aus dem wenigstens einen zu dehydrierenden Kohlenwasserstoff und nur molekularem Sauerstoff oder molekularem Sauerstoff und einem oder mehreren Inertgasen bestehen. Selbstverständlich kann im Rahmen des erfindungsgemäßen Verfahrens molekularen Sauerstoff enthaltendes Gas auch sowohl vorab des Eintritts des Reaktionsgases in die Reaktionszone als auch längs des Reaktionspfades in der Reaktionszone dem Reaktionsgas zugesetzt werden.

Erfindungsgemäß bevorzugt ist es, dem Reaktionsgas die Gesamtmenge an molekularem Sauerstoff enthaltendem Gas vorab des Eintritts in die Reaktionszone zuzusetzen. Dabei ist es, wie beim erfindungsgemäßen Verfahren ganz allgemein, von Vorteil, wenn dem Reaktionsgas weder vorab des Eintritts in die Reaktionszone noch längs des Reaktionspfades externer molekularer Wasserstoff zugesetzt wird.

In der Regel beträgt die dem Reaktionsgas vorab des Eintritts in die Reaktionszone zugesetzte Menge an molekularem Sauerstoff, bezogen auf die Gesamtmenge des zu dehydrierenden Kohlenwasserstoffes (z.B. Propan) 0,001 bis 0,5 mol/mol, bevorzugt 0,005 bis 0,2 mol/mol und besonders bevorzugt 0,05 bis 0,2 mol/mol. Die höheren Verhältnisse werden für höhere Propanumsätze bevorzugt angewendet. Erfindungsgemäß zweckmäßig wird die Menge der dem Reaktionsgas zugesetzten Menge an molekularem Sauerstoff so bemessen, dass längs des Reaktionspfades durch die Verbrennung von molekularem Wasserstoff die für die heterogen katalysierte Dehydrierung des wenigstens einen Kohlenwasserstoff benötigte Wärmemenge im wesentlichen erzeugt wird. In besonderen Ausführungsformen kann die durch die Verbrennung von molekularem Wasserstoff mit molekularem Sauerstoff erzeugte Wärme auch größer oder kleiner sein als die für die Dehydrierung des Kohlenwasserstoffs benötigte Wärme.

Erfindungsgemäß zweckmäßig wird dem Reaktionsgas die Gesamtmenge an benötigtem Inertgas (ausnähmlich von in der Reaktionszone sich durch chemische Reaktion bildendem Inertgas) ebenfalls vorab des Eintritts in die Reaktionszone zugesetzt. D.h., erfindungsgemäß wird keine Inertgaszwischeneinspeisung vorgenommen.

Mit Vorteil wird beim erfindungsgemäßen Verfahren als inertes Verdünnungsgas Wasserdampf mitverwendet. Wasserdampf bedingt neben seiner verdünnenden Wirkung als weiterem Vorteil in der Regel ein vermindertes Verkoken der für das erfindungsgemäße Verfahren eingesetzten Katalysatoren, da der Wasserdampf mit solchermaßen gebildetem Koks normalerweise nach dem Prinzip der Kohlevergasung abreagiert.

Erfindungsgemäß bevorzugt wird ebenfalls keine Wasserdampfzwischeneinspeisung vorgenommen, sondern dem Reaktionsgas vorab des Eintritts in die Reaktionszone die zuzuführende Wasserdampfmenge auf einmal zugesetzt. Das Verhältnis von Wasserdampf zu dem wenigstens einen zu dehydrierenden Kohlenwasserstoff beträgt im Reaktionsgas vorab seines Eintritts in die Reaktionszone daher im allgemeinen 0 bis 10 mol/mol, häufig 0,1 bis 10 mol/mol und bevorzugt 0,1 bis 5 mol/mol.

Da der im Rahmen des erfindungsgemäßen Verfahrens benötigte molekulare Sauerstoff normalerweise als Bestandteil von Luft zugesetzt wird, enthält das Reaktionsgas vorab seines Eintritts in die Reaktionszone normalerweise auch molekularen Stickstoff als inertes Verdünnungsgas.

D.h., in der Regel wird das beim erfindungsgemäßen Verfahren der Reaktionszone zugeführte Reaktionsgas Wasserdampf und Stickstoff als inerte Verdünnungsgase enthalten.

Kennzeichnendes Merkmal des erfindungsgemäßen Verfahrens ist es, daß das der Reaktionszone entnommene, molekularen Wasserstoff, Wasserdampf, den wenigstens einen zu dehydrierenden Kohlenwasserstoff und den wenigstens einen dehydrierten Kohlenwasserstoff enthaltende, Produktgas in zwei Teilmengen identischer Zusammensetzung aufgeteilt und eine der beiden Teilmengen als Reaktionsgas in die Reaktionszone zurückgeführt wird. Diese Rückführung kann sowohl in Form von Zwischeneinspeisungen als auch am Eintritt des Reaktionsgases in die Reaktionszone erfolgen. Erfindungsgemäß bevorzugt erfolgt sie ausschließlich am Eintritt des Reaktionsgases in die Reaktionszone. Das Reaktionsgas selbst wird beim erfindungsgemäßen Verfahren der Reaktionszone normalerweise lediglich an einer Stelle und nicht über zusätzliche Zwischeneinspeisungen zugeführt.

Die Summe aus der Reaktionszone zugeführtem Reaktionsgas und am Eintritt des Reaktionsgases in die Reaktionszone rückgeführtem Kreisgas soll in dieser Schrift als Beschickungsgas bezeichnet werden.

Die Vorteilhaftigkeit des erfindungsgemäßen Verfahrens wird darauf zurückgeführt, daß die in die Reaktionszone rückgeführte Produktgasmenge noch im Verlauf der heterogen katalysierten partiellen Dehydrierung gebildeten molekularen Wasserstoff enthält, was die Möglichkeit eröffnet, beim erfindungsgemäßen Verfahren auf den Zusatz von externem Wasserstoff gänzlich zu verzichten und dennoch die Vorteile der Wasserstoffverbrennung zu erzielen. Insbesondere wenn das in die Reaktionszone rückgeführte Kreisgas Bestandteil des Beschickungsgases ist, enthält das Beschickungsgas so von Anfang an für Verbrennungszwecke molekularen Wasserstoff, der nicht einer teuren externen Quelle entstammt.

Die Verbrennung des im Kreisgas enthaltenen molekularen Wasserstoff führt zu einer Wasserstoffeliminierung aus dem_Dehydriergleichgewicht, was unter sonst identischen Bedingungen eine Erhöhung des thermodynamisch möglichen Dehydrierumsatzes bewirkt. Darüberhinaus geht mit der erfindungsgemäßen Maßnahme der Kreisgasrückführung eine erhöhte Selektivität der Bildung von dehydriertem Kohlenwasserstoff einher. Dies gilt insbesondere dann, wenn es sich bei dem zu dehydrierenden Kohlenwasserstoff um Propan handelt. Mit ursächlich für diese Selektivitätserhöhung dürfte sein, daß der im Kreisgas in die Reaktionszone rückgeführte, bereits einmal dehydrierte, Kohlenwasserstoff sich bezüglich der angestrebten vergleichsweise spezifischen katalytischen Dehydrierung sowie Wasserstoffverbrennung in überraschender Weise weitgehend inert verhält.

Erfindungsgemäß zweckmäßig wird man wenigstens 10 % bzw. 20 % des der Reaktionszone entnommenen Produktgases in die Reaktionszone rückführen. Erfindungsgemäß vorteilhaft wird die als Kreisgas in die Reaktionszone rückgeführte Teilmenge des der Reaktionszone entnommenen Produktgases jedoch nicht mehr als 90 % bzw. 80 % betragen. D.h., die als Kreisgas in die Reaktionszone rückgeführte Teilmenge des der Reaktionszone entnommenen Produktgases kann beim erfindungsgemäßen Verfahren z.B. 20 bis 80 %, oder 30 bis 70 %, oder 40 bis 60 % oder auch 50 % der entnommenen Produktmenge betragen. Besonders vorteilhaft ist der Mengenbereich 50 bis 70 %.

Werden beim erfindungsgemäßen Verfahren in bevorzugter Weise das Reaktionsgas und das Kreisgas der Reaktionszone jeweils nur an einer Stelle so zugeführt, daß das Beschickungsgas sowohl die der Reaktionszone insgesamt zugeführte Menge an molekularem Sauerstoff als auch an molekularem Wasserstoff enthält, kann man durch geeignete Wahl des molaren Verhältnisses von molekularem Sauerstoff zu molekularem Wasserstoff im Beschickungsgas den Gesamtverlauf der erfindungsgemäßen heterogen katalysierten partiellen Dehydrierung wenigstens eines zu dehydrierenden Kohlenwaserstoffes, bezogen auf einmaligen Durchgang des Beschickungsgasgemisches durch die Reaktionszone, sowohl endotherm, autotherm (die Bruttoreaktionsenthalpie über die Reaktionszone ist im wesentlichen Null) oder exotherm verwirklichen. Eine autotherme bis leicht exotherme Fahrweise ist erfindungsgemäß bevorzugt. Als erfindungsgemäß günstig wird es in diesem Fall erachtet, wenn das vorgenannte molare Verhältnis von im Beschickungsgas befindlichem molekularem Sauerstoff zu im Beschickungsgas befindlichem molekularem Wasserstoff 1:1 oder kleiner und besonders bevorzugt 1:2 und kleiner beträgt. Wählt man das vorgenannte Verhältnis größer als 1:2 und führt man vom der Reaktionszone entnommenen Produktgas mehr als 90 % als Kreisgas, kann man die thermodynamische Kontrolle der erfindungsgemäßen heterogen katalysierten partiellen Dehydrierung weitgehend auflösen und Umsätze im Bereich von 80 bis 90 mol%, bezogen auf einmaligen Durchgang des Reaktionsgases, erzielen. In der Regel ist der Gesamtreaktionsverlauf dann exotherm.

Je nach Bedarf kann das Beschickungsgas der Reaktionszone bereits vorerwärmt zugeführt werden.

Ohne besondere Maßnahmen kann das erfindungsgemäße Verfahren so gestaltet werden, daß bei einmaligem Durchgang des Reaktionsgases durch die Reaktionszone ein Umsatz des zu dehydrierenden Kohlenwasserstoff (z.B. Propan oder Butan) von ≥ 5 mol-% bis ≤ 70 mol-% erzielt wird.

Die Wahl des Umsatzes orientiert sich insbesondere an der Folgeverwendung des nicht als Kreisgas in die Reaktionszone rückgeführten Teils des der Reaktionszone entnommenen Produktgases. Dieser Produktaustrag kann z.B. in an sich bekannter Weise dadurch aufgearbeitet werden, daß man die in ihm enthaltenen, von Kohlenwasserstoff verschiedenen, Bestandteile beispielsweise durch selektive Absorption der enthaltenen Kohlenwasserstoffe in einem organischen Lösungsmittel, z.B. wie in der DE-A 10 028 582 beschrieben, und/oder rektifikativ abtrennt und das verbliebene Gemisch aus zu dehydrierendem Kohlenwasserstoff und dehydriertem Kohlenwasserstoff in einem Splitter (z.B. im Fall von Propan als zu dehydrierenden Kohlenwasserstoff) auftrennt und den Anteil des zu dehydrierenden Kohlenwasserstoff als Bestandteil des Reaktionsgases in die Dehydrierung rückführt, während der abgetrennte dehydrierte Kohlenwasserstoff einer weiteren Zielumsetzung zugeführt wird. Für diesen Fall wird man das erfindungsgemäße Verfahren zweckmäßig im Bereich höherer Kohlenwasserstoffumsätze (typrisch 35 bis 60 mol-%) durchführen.

Häufig wird man das erfindungsgemäße Verfahren jedoch mit einem Verfahren zur Herstellung von partiellen Oxidationsprodukten und/ oder partiellen Ammoxidationsprodukten oder mit einem Verfahren zur Herstellung von Alkylierungsprodukten, Diels-Alder Addukten, Oxichlorierungsproudkten und Methatheseprodukten des wenigstens einen, im der Reaktionszone des erfindungsgemäßen Verfahrens entnommenen Produktgas enthaltenen, dehydrierten Kohlenwasserstoff (z.B. Propen oder iso-Buten) koppeln, wie es z.B. in der US-A 3 161 670, der EP-A 117 146, der DE-A 33 13 573, der DE-A 10 028 582 sowie der DE-A 10 131 297 oder in der DE-A 10 148 575, der DE-A 10 148 577, der DE-A 10 206 954, der DE-A 10 159 615, der DE-A 10 118 634, der DE-A 10 130 958 und der DE-A 10 160 726 empfohlen wird. In diesen Fällen sind Umsätze beim erfindungsgemäßen Dehydrierverfahren von ≥ 5 mol-% bis ≤ 30 mol-% oder ≤ 25 mol-%, häufig etwa 20 mol-%, ausreichend.

Dazu wird man aus der Teilmenge (Produktgasgemisch A) des beim erfindungsgemäßen Verfahren der Reaktionszone entnommenen Produktgases, das nicht in die Reaktionszone rückgeführt wird, von den darin enthaltenen, von dem wenigstens einen zu dehydrierenden Kohlenwasserstoff (z.B. Propan, n-Butan oder iso-Butan) und dem wenigstens einen dehydrierten Kohlenwasserstoff (z.B. Propen, n-Buten oder iso-Buten) verschiedenen Bestandteilen gegebenenfalls in einer Abtrennzone eine Teil- oder die Gesamtmenge unter Erhalt eines Produktgasgemisches A' abtrennen und entweder das Produktgasgemisch A oder das Produktgasgemisch A' zur Beschickung einer der vorgenannten Nachfolgereaktionen wie z.B. einer heterogen katalysierten Oxidations- und/oder Ammoxidationszone verwenden, in der der enthaltene wenigstens eine dehydrierte Kohlenwasserstoff z.B. einer partiellen Oxidation- und/oder Ammoxidation mit molekularem Sauerstoff unter Bildung eines Produktgemisches B unterworfen wird, das als Zielprodukt wenigstens ein partielles Oxidations- und/oder Ammoxidationsprodukt (z.B. Acrylsäure, Methacrylsäure, Acrolein, Acrylnitril etc.) des wenigstens einen dehydrierten Kohlenwasserstoff enthält.

In einer Aufarbeitungszone C wird anschließend aus dem Produktgasgemisch B Zielprodukt abgetrennt und vom im restlichen Produktgasgemisch B enthaltenen nicht umgesetzten wenigstens einen zu dehydrierenden Kohlenwasserstoff wenigstens eine Teilmenge als Bestandteil eines zweiten Kreisgases in die Reaktionszone des erfindungsgemäßen Dehydrierverfahrens als Quelle für das Reaktionsgas rückgeführt.

Dieses zweite Kreisgas wird in der Regel neben dem zu dehydrierenden Kohlenwasserstoff häufig noch inerte und/oder nicht inerte Verdünnungsgase wie Oxygenate, Methan, Ethan, Ethylen, höhere Kohlenwasserstoffe, Wasserdampf, N₂, CO, CO₂ und gegebenenfalls molekularen Sauerstoff enthalten.

Im Fall einer solchen Kopplung des erfindungsgemäßen Verfahrens mit einer heterogen katalysierten partiellen Oxidation und/oder Ammoxidation des wenigstens einen dehydrierten Kohlenwasserstoff ist es vorteilhaft, das Oxidations- und/oder Ammoxidationsverfahren in Anwendung eines Überschusses an molekularem Sauerstoff bezüglich der Oxidations- und/oder Ammoxidationsstöchiometrie durchzuführen. In zweckmäßiger Weise wird man in der Aufarbeitungszone C dann so arbeiten, daß dieser Überschuß an molekularem Sauerstoff in dem zweiten Kreisgas im wesentlichen verbleibt. Dieses molekularen Sauerstoff enthaltende zweite Kreisgas kann dann als Bestandteil des Reaktionsgases für das erfindungsgemäße Verfahren Quelle (bevorzugt alleinige Quelle) des im Rahmen des erfindungsgemäßen Verfahrens benötigten molekularen Sauerstoff sein. Das Vorgenannte gilt insbesondere dann, wenn der zu dehydrierende Kohlenwasserstoff Propan und das partielle Oxidationsprodukt des dehydrierten Kohlenwasserstoff Propen das Acrolein und/oder die Acrylsäure ist.

Enthält das in die Reaktionszone des erfindungsgemäßen Verfahrens rückgeführte noch molekularen Sauerstoff enthaltende zweite Kreisgas auch noch CO, Oxygenate wie z.B. Acrolein, Acrylsäure, Formaldehyd, Essigsäure und/oder Ethylenoxid, so können diese vorab und/oder in dieser Reaktionszone mit dem molekularen Sauerstoff gleichfalls heterogen katalysiert zu z.B. CO₂ und H₂O aufoxidiert werden. Die dabei frei werdende Reaktionswärme kann ebenfalls dem Zweck der Erwärmung des Reaktionsgases dienen.

Erfindungsgemäß vorteilhaft umfaßt die Reaktionszone des erfindungsgemäßen Verfahrens lediglich eine Katalysatorschüttung, wobei als Katalysator ein solcher gemäß Beispiel 1, oder gemäß Beispiel 2 oder gemäß Beispiel 3, oder gemäß Beispiel 4 der DE-A 19 937 107 oder gemäß Ausführungsbeispiel der DE-A 10 060 099 eingesetzt wird.

Ist der zu dehydrierende Kohlenwasserstoff Propan und der dehydrierte Kohlenwasserstoff Propen, so kann sich das Beschickungsgasgemisch damit zusammensetzen aus:
a) 50 bis 70 % des der (Dehydrier)Reaktionszone entnommenen Produktgases (erstes Kreisgas).
Dieses Produktgas ist üblicherweise wie folgt zusammengesetzt:

| | |
|---|---|
| 0 - 1 Vol.-% Methan, | 0 - 60 Vol.-% H₂O, |
| 0 - 1 Vol.-% Ethan, | 0 - 1 Vol.-% C₄₊-Kohlenstoff, |
| 0 - 1 Vol.-% Ethen, | 0 - 2 Vol.-% CO₂, |
| 1 - 10 Vol.-% H₂, | 0 - 2 Vol.-% O₂, |
| 1 - 20 Vol.-% Propen, | 10 - 40 Vol.-% Propan, |
| 0 - 1 Vol.-% CO, | Rest bis 100 Vol.-%: im wesentlichen Stickstoff. |

b) einem zweiten Kreisgas, das aus der heterogen katalysierten Partialoxidation zu Acrolein und/oder Acrylsäure des im nicht direkt in die Dehydrierreaktionszone rückgeführten Teil es der Dehydrierreaktionszone entnommenen Produktgases enthaltenen Propen herrührt; dieses zweite Kreisgas ist üblicherweise wie folgt zusammengesetzt:

| | |
|---|---|
| 0 - 1 Vol.-% Methan, | 0 - 1 Vol.-% CO, |
| 0 - 5 Vol.-% H₂O, | 0 - 3 Vol.-% CO₂, |
| 0 - 1 Vol.-% Ethen, | 0 - 10 Vol.-% O₂, |
| 0 - 1 Vol.-% Ethan, | 10 - 40 Vol.-% Propan, |
| 0 - 1 Vol.-% Wasserstoff, | 0 - 1 Vol.-% C₄₊-Kohlenwasserstoffe, |
| 0 - 1 Vol.-% Propen, | Rest bis 100 Vol.-%: im wesentlichen Stickstoff. |

c) Frischpropan und frischem Wasserdampf.

Eine Zufuhr weiterer Gase in die Reaktionszone ist erfindungsgemäß nicht erforderlich.

Die Volumenverhältnisse betragen dabei zweckmäßig:
erstes Kreisgas: zweitem Kreisgas: Frischpropan: Frischwasserdampf = 10-30 : 10-30 : 1 : 0,1-5.

Bevorzugt betragen vorgenannte Volumenverhältnisse 20:20:1:1. Die heterogen katalysierte Dehydrierung wird dabei bevorzugt im wesentlichen autotherm bei einer Temperatur von 450°C bis 650°C und bei einem Druck von 1 bar bis 3 bar durchgeführt. Die Belastung des Dehydrierkatalysators mit Beschickungsgas beträgt zweckmäßig 2000 bis 20 000 Nl/l Kat. · h.

Bevorzugte Katalysatorgeometrie sind Stränge der Länge 2 mm bis 10 mm und eines Durchmessers des Querschnitts von 1 mm bis 4 mm.

Der verwendete Katalysator kann wie in der DE-A 10 028 582 beschrieben regeneriert werden. Bei ausgeprägter Desaktivierung kann die Regenerierprozedur mehrfach hintereinander angewendet werden, um die Ursprungsperformance im wesentlichen wieder rückzugewinnen. Die Regenerierprozedur enthält einen Regenerierschritt mit reinem oder mit durch Inertgas verdünntem molekularem Wasserstoff. Um die für diesen Regenerierschritt benötigte Wasserstoffmenge zu beschränken, ist es anwendungstechnisch zweckmäßig, den Regenerierwasserstoff entweder im Kreis zu fahren oder in besonders einfacher Weise längere Zeit über dem deaktivierten Katalysator stehen zu lassen. Als Reaktor kann für die Dehydrierung ein einfacher Schachtreaktor verwendet werden. Die Partialoxidation kann wie in den zitierten Schriften des Standes der Technik (insbesondere die DE-A 10 311 297 und die DE-A 10 028 582) beschrieben realisiert werden. Dabei wird man aus dem Produktgasgemisch A vorab seiner Verwendung für die Partialoxidation bevorzugt alle von Propan und Propen verschiedenen Bestandteile abtrennen.

Üblicherweise wird beim erfindungsgemäßen Verfahren die Kreisgasrückführung in die Reaktionszone mittels eines außerhalb der Reaktionszone liegenden Kompressors durchgeführt.

In besonders eleganter Weise läßt sich diese Kreisgasführung jedoch in einem Strahlpumpen-Kreislaufreaktor realisieren.

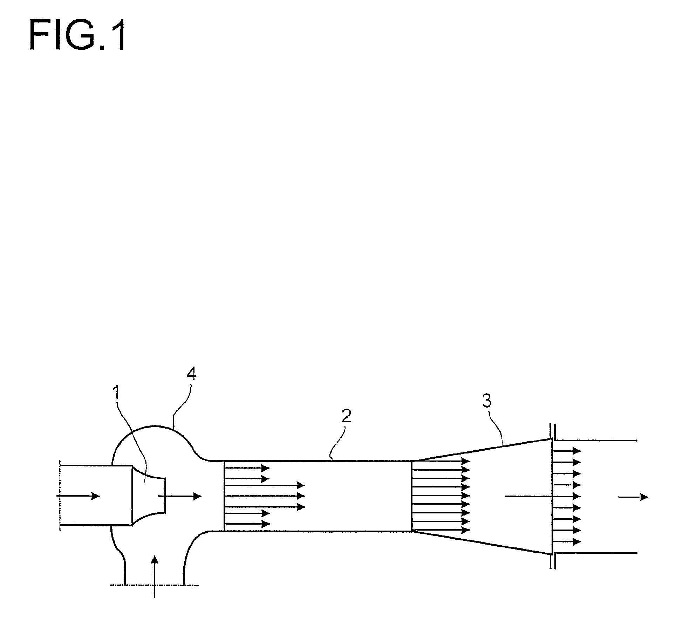

Ein solcher Reaktor umfaßt die Katalysatorbeschickung und wenigstens eine Strahlpumpe (Figur 1 zeigt die schematische Skizze einer Strahlpumpe). Letztere umfaßt eine Treibdüse (1), ein Mischrohr (2), einen Diffusor (3) und einen Saugstutzen (4). Die Ziffern in Klammern beziehen sich auf die Figur 1.

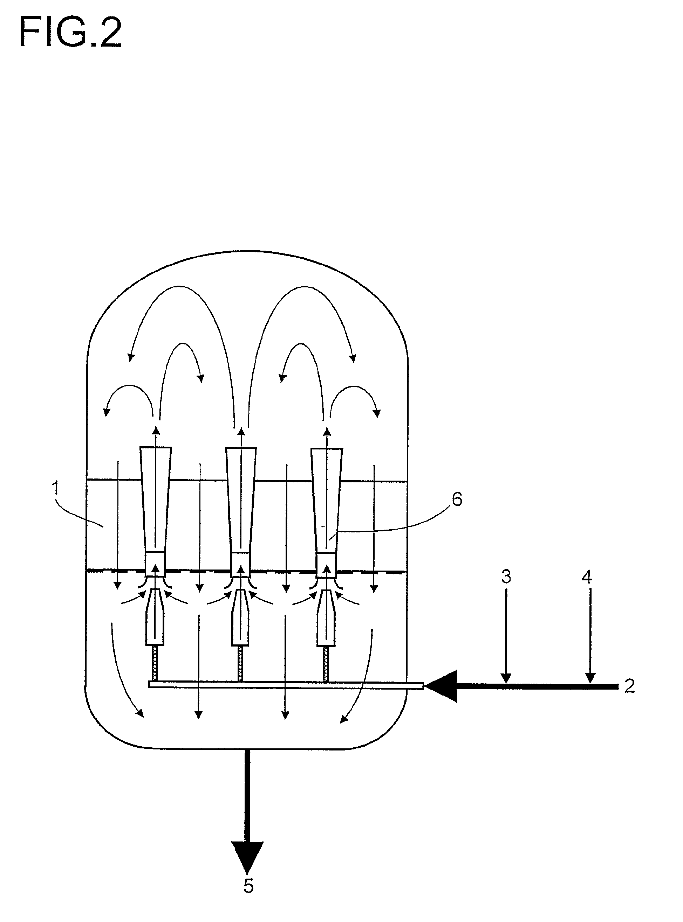

Durch die Treibdüse wird in Förderrichtung ein Treibstrahl in das Mischrohr entspannt. Im Saugstutzen wird dadurch ein Unterdruck erzeugt, durch das den Saugstutzen umgebendes Gas angesaugt und bei gleichzeitiger Vermischung mit dem Treibstrahl durch das Mischrohr über den Diffusor transportiert und entlassen wird. Der Treibstrahl wird beim erfindungsgemäßen Verfahren in zweckmäßiger Weise durch das der Reaktionszone zuzuführende Reaktionsgas gebildet. Hinter dem Diffusor wird die Förderrichtung umgekehrt und das Reaktionsgas der Katalysatorbeschickung zugeführt. Eine Teilmenge des die Katalyssatorbeschickung verlassenden Produktgases wird über den Saugstutzen partiell angesaugt, im Mischrohr mit Reaktionsgas vermischt und über den Diffusor als Beschickungsgasgemisch entlassen. Die Restmenge des Produktgases wird aus dem Reaktor geführt. Der in dieser Schrift verwendete Begriff der Strahlpumpe soll auch Ejektorstrahldüsen umfassen. Eine beispielhafte Gestaltung eines Strahlpumpen-Kreislaufreaktors zeigt die Figur 2. In der Figur 2 haben die Ziffern folgende Bedeutung:
1 = Katalysatorbett; 2 = z.B. Propan; 3 = z.B. Luft; 4 = z.B. Wasserdampf; 5 = Produktgas; 6 = Strahlpumpen.

Es handelt sich dabei um einen Strahlpumpen-Kreislaufreaktor des Typs, bei dem sich der Saugstutzen auf der einen und der Diffusor auf der anderen Seite der Katalysatorbeschickung befindet. Bei im Reaktorinneren liegenden Strahlpumpen kann es zweckmäßig sein, den Saugstutzen verschließbar zu gestalten. Dies kann insbesondere für Zwecke der Regenerierung der Katalysatorbeschickung von Vorteil sein. Dabei wird der Treibstrahl durch Regeneriergas gebildet während der Saugstutzen verschlossen ist. Er kann aber auch geöffnet sein. Selbstredend kann sich beim Strahlpumpen-Kreislaufreaktor die wenigstens eine Strahlpumpe auch außerhalb des die Katalysatorbeschickung enthaltenden Reaktorraumes befinden.

Ein noch zu erwähnender weiterer Vorteil der erfindungsgemäßen Verfahrensweise besteht in einer verlängerten Standzeit der Katalysatorbeschickung im Vergleich zu einer Fahrweise ohne Kreisgasrückführung in die Reaktionszone.

### Beispiele und Vergleichsbeispiele

### 1. Präparierung des Dehydrierreaktors

1000 g eines gesplitteten ZrO₂ · Si02 Mischoxids wurden mit einer Lösung von 11,993 g SnCl₂ · 2 H₂O und 7,886 g H₂PtCl₆ · 6 H₂O in 600 ml Ethanol übergossen.

Das Mischoxid wies ein ZrO₂/SiO₂-Gewichtsverhältnis von 95:5 auf. Hersteller des Mischoxids war die Firma Norton (USA).

Das Mischoxid wies folgende Spezifikation auf:
Type AXZ 311070306, Lot-No. 2000160042, Siebfraktion: 1,6 bis 2 mm, BET-Oberfläche: 86 m²/g, Porenvolumen: 0,28 ml/g (Quecksilber-Porosimetrie-Messung).

Das überstehende Ethanol wurde am Rotavapor unter Rotieren im Wasserstrahlpumpenvakuum (20 mbar) bei einer Wasserbadtemperatur von 40°C abgezogen. Anschließend wurde jeweils unter stehender Luft zunächst 15 h bei 100°C getrocknet und anschließend bei 560°C während 3 h calciniert. Danach wurde der getrocknete Feststoff mit einer Lösung von 7,71 g CsNO₃, 13,559 g KNO₃ und 98,33 g La(NO₃)₃ · 6 H₂O in 2500 ml H₂O übergossen. Das überstehende Wasser wurde am Rotavapor unter Rotieren im Wasserstrahlpumpenvakuum (20 mbar) bei einer Wassertemperatur von 85°C abgezogen. Anschließend wurde jeweils unter stehender Luft zunächst 15 h bei 100°C getrocknet und anschließend bei 560°C während 3 h calciniert.

Der so erhaltene Katalysatorvorläufer wies eine Zusammensetzung von Pt_{0,3}Sn_{0,6}Cs_{0,5}K_{0,5}La_{3,0} (Gewichtsverhältnisse) auf (ZrO₂)₉₅ · (SiO₂)₅ (Gewichtsverhältnis) auf.

Mit 20 ml des erhaltenen Katalysatorläufers wurde ein vertikal stehender Rohrreaktor beschickt (Reaktorlänge: 800 mm; Wanddicke: 2 mm; Innendurchmesser: 20 mm; Reaktormaterial: innen-alonisiertes (d.h. mit Aluminiumoxid beschichtetes) Stahlrohr; Beheizung: elektrisch (Ofen der Fa. HTM Reetz, LOBA 1100-28-650-2) auf einer längsmittigen Länge von 650 mm; Länge der Katalysatorschüttung: 75 mm; Position der Katalysatorschüttung: auf der Längsmitte des Rohrreaktors; Befüllung des Reaktorrestvolumens nach oben und unten mit Steatitkugeln (Inertmaterial) eines Durchmessers von 4 - 5 mm, nach unten auf einem Kontaktstuhl aufsitzend). Anschließend wurde das Reaktionsrohr bei einer Außenwandtemperaturregelung längs der Heizzone von 500°C (bezogen auf ein mit einem identischen Inertgasstrom durchströmtes Rohr) mit 9,3 Nl/h Wasserstoff während 30 min beschickt. Anschließend wurde der Wasserstoffstrom bei gleichbleibender Wandtemperatur zunächst während 30 min durch einen 23,6 Nl/h betragenden Strom aus 80 Vol.-% Stickstoff und 20 Vol.-% Luft und danach während 30 min durch einen identischen Strom aus reiner Luft ersetzt. Unter Beibehalt der Wandtemperatur wurde dann mit einem identischen Strom von N₂ während 15 min gespült und abschließend nochmals während 30 min mit 9,3 Nl/h Wasserstoff reduziert. Damit war die Aktivierung des Katalysatorvorläufers abgeschlossen.

### 2. Vergleichsbeispiel 1

Ein wie unter 1. beschrieben präparierter Dehydrierreaktor wurde bei gleichbleibender Temperaturregelung (500°C Wandtemperatur bezogen auf Inertgasdurchströmung) mit einem Gemisch aus 20 Nl/h Roh-Propan und 20 g/h Wasserdampf als Reaktionsgas beschickt. Dabei wurde das Roh-Propan mittels eines Massendurchflussreglers der Fa. Brooks zudosiert, während das Wasser mittels einer HPLC Pump 420 der Fa. Kontron zunächst flüssig in einen Verdampfer dosiert, in selbigem verdampft und dann mit dem Roh-Propan vermischt wurde. Die Temperatur des Reaktionsgases betrug 150°C.

Mittels einer sich am Reaktorausgang befindlichen Druckregelung (Fa. REKO) wurde der Ausgangsdruck des Reaktors auf 1,5 bar absolut eingestellt.

Das hinter der Druckregelung auf Normaldruck entspannte Produktgas wurde abgekühlt, wobei der enthaltene Wasserdampf auskondensierte. Das nicht kondensierte Restgas wurde mittels GC (HP 6890 mit Chem-Station, Detektoren: FID, WLD, Trennsäulen: Al₂O₃/KCl (Chrompack), Carboxen 1010 (Supelco)) analysiert. In entsprechender Weise war auch das Reaktionsgas analysiert worden.

Die nachfolgende Tabelle 1 weist die erzielten Ergebnisse in Abhängigkeit von der Betriebsdauer aus. Dabei beziehen sich die Angaben in Vol.-% auf "trocken" gerechnetes Gas. D.h., die enthaltene Menge an Wasserdampf blieb in allen Fällen unberücksichtigt.

**Tabelle 1**

| | Reaktionsgas Vol.-% | Produktgas (nach 1 h) Vol.-% | Produktgas nach (9 h) Vol.-% |
|---|---|---|---|
| Propan | 99,99 | 64,25 | 62,81 |
| Propen | 0,0096 | 16,61 | 16,49 |
| H₂ | 0 | 18,74 | 20,33 |
| O₂ | 0 | 0 | 0 |
| N₂ | 0 | 0,068 | 0,065 |
| Methan | 0 | 0,040 | 0,033 |
| Ethan | 0,0017 | 0,118 | 0,108 |
| Ethen | 0 | 0,019 | 0,019 |
| CO | 0 | 0,019 | 0,014 |
| CO₂ | 0 | 0,129 | 0,124 |

Diesen Werten entspricht ein auf einmaligen Durchgang des Reaktionsgases bezogener Propanumsatz von 20,8 mol-% und eine Selektivität der Propenbildung von 99,1 mol-%.

### Vergleichsbeispiel 2

Die Randbedingungen des Vergleichsbeispiels 1 wurden beibehalten, dem Reaktionsgas wurden jedoch zusätzlich 7,5 Nl/h Luft zugesetzt und der Versuch anschließend weitergeführt. Die Propanbelastung des Katalysatorbettes entsprach jener aus Vergleichsbeispiel 1. Die nachfolgende Tabelle 2 weist die erhaltenen Ergebnisse in Abhängigkeit von der Betriebsdauer (Zeitdauer ab Umstellung auf die neuen Reaktionsbedingungen) aus. Die Angaben in Vol.-% beziehen sich wieder auf "trocken gerechnetes" Gas.

**Tabelle 2**

| | Reaktionsgas Vol.-% | Produktgas (nach 3 h) Vol.-% | Produktgas nach (20 h) Vol.-% |
|---|---|---|---|
| Propan | 73,16 | 45,43 | 46,49 |
| Propen | 0,007 | 14,40 | 14,03 |
| H₂ | 0 | 17,56 | 17,01 |
| O₂ | 5,34 | 0 | 0 |
| N₂ | 21,50 | 19,28 | 18,97 |
| Methan | 0 | 0,16 | 0,24 |
| Ethan | 0,0012 | 0,20 | 0,22 |
| Ethen | 0 | 0,05 | 0,07 |
| CO | 0 | 0,16 | 0,16 |
| CO₂ | 0 | 2,77 | 2,81 |

Ein Vergleich mit Tabelle 1 weist aus, daß das Beisein des Luftsauerstoff sowohl eine Erhöhung der COₓ-Bildung als auch eine Verstärkung der Bildung von Crackprodukten (Methan, Ethan und Ethen) bewirkt. Beides weist eine geringere Selektivität der Propenbildung aus. Letztere wurde, bezogen auf einen einmaligen Durchgang der Reaktionsgases, bei einem Propanumsatz von 24,6 mol-% als 91,4 mol-% ermittelt.

### Ausführungsbeispiel

Die Randbedingungen des Vergleichsbeispiels 2 wurden beibehalten, allerdings wurden 422 l/h des auf 150°C abgekühlten Produktgases an den Reaktoreingang zurückgeführt (mittels eines Membran-Kompressors der Fa. KNF Neuberger, P ≈ 1,5 bar) und dort mit dem Reaktionsgas gemäß Vergleichsbeispiel 2 zum der Katalysatorschüttung zugeführten Beschickungsgas vermischt. Die Einstellung der Kreisgasmenge erfolgte mittels einer mit dem Membran-Kompressor gekoppelten Blendenmessung (Fa. Rosemount, Blendendurchmesser: 1,3 mm). Um ein Auskondensieren von Wasser zu vermeiden, wurden der Membran-Kompressor, die Blendmessung und die Kreisgasleitungen mittels Heizbändern auf 150°C beheizt. Die Propanbelastung aus Vergleichsbeispiel 2 wurde beibehalten. Der Teil des Produktgases, der nicht im Kreis in die Reaktionszone rückgeführt wurde, wurde wie beschrieben analysiert. Die nachfolgende Tabelle 3 weist die erhaltenen Ergebnisse in Abhängigkeit von der Betriebsdauer (Zeitdauer ab Umstellung auf die neuen Reaktionsbedingungen) aus. Die Angaben in Vol.-% beziehen sich wieder auf "trocken gerechnetes" Gas.

**Tabelle 3**

| | Beschickungsgas Vol.-% | Produktgas (nach 3 h) Vol.-% | Produktgas nach (20 h) Vol.-% |
|---|---|---|---|
| Propan | 73,08 | 60,15 | 57,97 |
| Propen | 0,007 | 13,10 | 14,51 |
| H₂ | 0 | 5,36 | 6,94 |
| O₂ | 5,36 | 0 | 0 |
| N₂ | 21,55 | 20,73 | 19,93 |
| Methan | 0 | 0,04 | 0,04 |
| Ethan | 0,0009 | 0,12 | 0,13 |
| Ethen | 0 | 0,12 | 0,11 |
| CO | 0 | 0,01 | 0,02 |
| CO₂ | 0 | 0,37 | 0,36 |

Der Vergleich von Tabelle 3 mit der Tabelle 2 zeigt, dass die erfindungsgemäße Kreisgasfahrweise die in Vergleichsbeispiel 2 durch die Luftzugabe bedingte Zunahme der COₓ-Bildung und die Entstehung von Crackprodukten (Methan, Ethan und Ethen) bezogen auf gebildetes Propen auf ein Niveau zurückdrängt, das nahezu jenem in Vergleichsbeispiel 1, das unter Sauerstoffausschluß durchgeführt wurde, entspricht. Dies weist eine Zunahme der Selektivität der Propenbildung gegenüber Vergleichsbeispiel 2 aus. Weiterhin weisen die numerischen Werte für den Wasserstoffgehalt im Vergleich mit Tabelle 2 aus, daß der rezirkulierte Wasserstoff weitgehend selektiv durch den Lauftsauerstoff oxidiert worden ist. Bezogen auf einmaligen Durchgang des Beschickungsgases betrug die Selektivität der Propenbildung 98,0 mol-% bei einem Propanumsatz von 20,3 mol-%.

## Patentansprüche

1. Verfahren der kontinuierlichen heterogen katalysierten partiellen Dehydrierung wenigstens eines zu dehydrierenden Kohlenwasserstoffs in der Gasphase, bei dem
- einer Reaktionszone ein den wenigstens einen zu dehydrierenden Kohlenwasserstoff enthaltendes Reaktionsgas kontinuierlich zugeführt wird,
- das Reaktionsgas in der Reaktionszone über wenigstens ein Katalysatorfestbett geführt wird, an welchem durch katalytische Dehydrierung molekularer Wasserstoff und partiell wenigstens ein dehydrierter Kohlenwasserstoff gebildet werden,
- dem Reaktionsgas vor und/oder nach Eintritt in die Reaktionszone wenigstens ein molekularen Sauerstoff enthaltendes Gas zugesetzt wird,
- der molekulare Sauerstoff in der Reaktionszone im Reaktionsgas enthaltenen molekularen Wasserstoff teilweise zu Wasserdampf oxidiert und
- der Reaktionszone ein Produktgas entnommen wird, das molekularen Wasserstoff, Wasserdampf, den wenigstens einen dehydrierten Kohlenwasserstoff und den wenigstens einen zu dehydrierenden Kohlenwasserstoff enthält,
**dadurch gekennzeichnet, daß** das der Reaktionszone entnommene Produktgas in zwei Teilmengen identischer Zusammensetzung aufgeteilt und eine der beiden Teilmengen als Kreisgas in die Reaktionszone zurückgeführt wird.

2. Verfahren nach Anspruch 1, **dadurch gekennzeichnet, daß** das wenigstens eine Katalysatorfestbett von Dehydrierkatalysatoren gebildet wird, die 10 bis 99,9 Gew.- % Zirkondioxid, 0 bis 60 Gew.- % Aluminiumoxid, Siliciumdioxid und/oder Titandioxid und 0,1 bis 10 Gew.- % mindestens eines Elements der ersten oder zweiten Hauptgruppe, eines Elements der dritten Nebengruppe, eines Elements der achten Nebengruppe des Periodensystems der Elemente, Lanthan und/oder Zinn mit der Maßgabe enthalten, daß die Summe der Gewichtsprozente 100 Gew.- % ergibt.

3. Verfahren nach Anspruch 1 oder 2, **dadurch gekennzeichnet, daß** das Katalysatorfestbett Katalysatorstränge und/oder Katalysatorringe umfaßt.

4. Verfahren nach einem der Ansprüche 1 bis 3, **dadurch gekennzeichnet, daß** der zu dehydrierende Kohlenwasserstoff Propan und/oder Butan ist.

5. Verfahren nach einem der Ansprüche 1 bis 4, **dadurch gekennzeichnet, daß** dem Reaktionsgas ausschließlich vor seinem Eintritt in die Reaktionszone wenigstens ein molekularen Sauerstoff enthaltendes Gas zugesetzt wird.

6. Verfahren nach einem der Ansprüche 1 bis 5, **dadurch gekennzeichnet, daß** als wenigstens ein molekularen Sauerstoff enthaltendes Gas Luft zugesetzt wird.

7. Verfahren nach einem der Ansprüche 1 bis 6, **dadurch gekennzeichnet, daß** der Reaktionszone abgesehen vom Kreisgas kein weiteres molekularen Wasserstoff enthaltendes Gas zugesetzt wird.

8. Verfahren nach einem der Ansprüche 1 bis 7, **dadurch gekennzeichnet, daß** das der Reaktionszone zugeführte Reaktionsgas Wasserdampf enthält.

9. Verfahren nach einem der Ansprüche 1 bis 8, **dadurch gekennzeichnet, daß** die als Kreisgas in die Reaktionszone rückgeführte Teilmenge des der Reaktionszone entnommenen Produktgases 20 bis 80 % der entnommenen Produktgasmenge beträgt.

10. Verfahren nach einem der Ansprüche 1 bis 9, **dadurch gekennzeichnet, daß** die nicht als Kreisgas in die Reaktionszone rückgeführte Teilmenge des Produktgases, gegebenenfalls nach einer Teilabtrennung von darin enthaltenen, von dem wenigstens einen dehydrierten Kohlenwasserstoff verschiedenen, Komponenten, zu Zwecken der partiellen Oxidation und/oder Ammoxidation des dehydrierten Kohlenwasserstoffs verwendet wird.

11. Verfahren nach einem der Ansprüche 1 bis 10, **dadurch gekennzeichnet, daß** das Reaktionsgas ein aus einer partiellen Oxidation und/oder Ammoxidation des dehydrierten Kohlenwasserstoffs kommendes, von dem wenigstens einen zu dehydrierenden Kohlenwasserstoff enthaltendes, zweites Kreisgas zugesetzt enthält.

12. Verfahren nach einem der Ansprüche 1 bis 11, **dadurch gekennzeichnet, daß** das aus der partiellen Oxidation und/oder Ammoxidation des dehydrierten Kohlenwasserstoffs in die Reaktionszone rückgeführte zweite Kreisgas das einzige molekularen Sauerstoff enthaltende Gas ist, das der Reaktionszone zugesetzt wird.

13. Verfahren nach einem der Ansprüche 1 bis 12, **dadurch gekennzeichnet, daß** es in einem Strahlpumpen- Kreislaufreaktor durchgeführt wird.

14. Reaktor, enthaltend wenigstens eine Katalysatorbeschickung, die sich zur katalytischen Dehydrierung von zu dehydrierenden Kohlenwasserstoffen eignet und wenigstens eine Strahlpumpe, wobei die Katalysatorbeschickung als Katalysatorfestbett ausgeführt ist und die wenigstens eine Strahlpumpe eine Treibdüse, ein Mischrohr, einen Diffusor und einen Saugstutzen umfasst.

## Claims

1. A process for the continuous heterogeneously catalyzed partial dehydrogenation of at least one hydrocarbon to be dehydrogenated in the gas phase, in which
- a reaction gas comprising at least one hydrocarbon to be dehydrogenated is fed continuously to a reaction zone,
- the reaction gas in the reaction zone is conveyed over at least one fixed catalyst bed over which molecular hydrogen and at least one dehydrogenated hydrocarbon are formed by catalytic (partial) dehydrogenation,
- at least one gas comprising molecular oxygen is added to the reaction gas before and/or after entry into the reaction zone,
- the molecular oxygen in the reaction zone oxidizes part of the molecular hydrogen present in the reaction gas to water vapor, and
- a product gas comprising molecular hydrogen, water vapor, the dehydrogenated hydrocarbon or hydrocarbons and the hydrocarbon or hydrocarbons to be dehydrogenated is taken from the reaction zone,
wherein the product gas taken from the reaction zone is divided into two substreams of identical composition and one of the two substreams is recirculated as circulated gas to the reaction zone.

2. The process according to claim 1, wherein the fixed catalyst bed or beds is made up of dehydrogenation catalysts comprising from 10 to 99.9% by weight of zirconium dioxide, from 0 to 60% by weight of aluminum oxide, silicon dioxide and/or titanium dioxide and from 0.1 to 10% by weight of at least one element of main group I or II, an element of transition group III, an element of transition group VIII of the Periodic Table of the Elements, lanthanum and/or tin, with the proviso that the percentages by weight add up to 100% by weight.

3. The process according to claim 1 or 2, wherein the fixed catalyst bed comprises catalyst extrudates and/or catalyst rings.

4. The process according to any of claims 1 to 3, wherein the hydrocarbon to be dehydrogenated is propane and/or butane.

5. The process according to any of claims 1 to 4, wherein at least one gas comprising molecular oxygen is added to the reaction gas exclusively before it enters the reaction zone.

6. The process according to any of claims 1 to 5, wherein air is added as at least one gas comprising molecular oxygen.

7. The process according to any of claims 1 to 6, wherein apart from the circulating gas no further gas comprising molecular hydrogen is introduced into the reaction zone.

8. The process according to any of claims 1 to 7, wherein the reaction gas fed to the reaction zone contains water vapor.

9. The process according to any of claims 1 to 8, wherein the substream of the product gas taken from the reaction zone which is recirculated as circulating gas to the reaction zone amounts to from 20 to 80% of the product gas taken off.

10. The process according to any of claims 1 to 9, wherein the substream of the product gas which is not recirculated as circulating gas to the reaction zone is, if appropriate after part of the components present therein other than the dehydrogenated hydrocarbon or hydrocarbons have been separated off, used for the partial oxidation and/or amoxidation of the dehydrogenated hydrocarbon.

11. The process according to any of claims 1 to 10, wherein the reaction gas further comprises an added second circulating gas which comprises the hydrocarbon or hydrocarbons to be dehydrogenated and comes from a partial oxidation and/or amoxidation of the dehydrogenated hydrocarbon.

12. The process according to any of claims 1 to 11, wherein the second circulating gas which comes from the partial oxidation and/or amoxidation of the dehydrogenated hydrocarbon and is recirculated to the reaction zone is the only gas comprising molecular oxygen which is added to the reaction zone.

13. The process according to any of claims 1 to 12 carried out in a jet pump circulation reactor.

14. A reactor comprising at least one catalyst bed which is suitable for the catalytic dehydrogenation of hydrocarbons to be dehydrogenated and at least one jet pump, the catalyst bed being designed as a fixed catalyst bed and the at least one jet pump comprises a driving nozzle, a mixing tube, a diffuser and a suction section.

## Revendications

1. Procédé pour la déshydrogénation partielle continue catalysée par voie hétérogène d'au moins un hydrocarbure à déshydrogéner en phase gazeuse, dans lequel
- une zone de réaction est alimentée en continu en un gaz de réaction contenant ledit au moins un hydrocarbure à déshydrogéner,
- le gaz de réaction est guidé dans la zone de réaction sur au moins un lit fixe catalytique sur lequel de l'hydrogène moléculaire et, en partie, au moins un hydrocarbure déshydrogéné sont formés par déshydrogénation catalytique,
- le gaz de réaction est additionné, avant et/ou après l'entrée dans la zone de réaction, d'au moins un gaz contenant de l'oxygène moléculaire,
- l'oxygène moléculaire oxyde partiellement l'hydrogène moléculaire contenu dans la zone de réaction, dans le gaz de réaction, en vapeur d'eau et
- un gaz produit est prélevé de la zone de réaction, qui contient de l'hydrogène moléculaire, de la vapeur d'eau, ledit au moins un hydrocarbure déshydrogéné et ledit au moins un hydrocarbure à déshydrogéner,
**caractérisé en ce que** le gaz produit prélevé de la zone de réaction est divisé en deux quantités partielles de composition identique et une des deux quantités partielles est recyclée en tant que gaz de circulation dans la zone de réaction.

2. Procédé selon la revendication 1, **caractérisé en ce que** ledit au moins un lit fixe catalytique est formé par des catalyseurs de déshydrogénation qui contiennent 10 à 99,9% en poids de dioxyde de zirconium, 0 à 60% en poids d'oxyde d'aluminium, de dioxyde de silicium et/ou de dioxyde de titane et 0,1 à 10% en poids d'au moins un élément du premier ou deuxième groupe principal, un élément du troisième groupe secondaire, un élément du huitième groupe secondaire du système périodique des éléments, du lanthane et/ou de l'étain, à condition que la somme des % en poids vaille 100% en poids.

3. Procédé selon la revendication 1 ou 2, **caractérisé en ce que** le lit fixe catalytique comprend des brins de catalyseur et/ou des anneaux de catalyseur.

4. Procédé selon l'une quelconque des revendications 1 à 3, **caractérisé en ce que** l'hydrocarbure à déshydrogéner est le propane et/ou le butane.

5. Procédé selon l'une quelconque des revendications 1 à 4, **caractérisé en ce que** le gaz de réaction est additionné, exclusivement avant son entrée dans la zone de réaction, d'au moins un gaz contenant de l'oxygène moléculaire.

6. Procédé selon l'une quelconque des revendications 1 à 5, **caractérisé en ce qu'**on ajoute de l'air comme au moins un gaz contenant de l'oxygène moléculaire.

7. Procédé selon l'une quelconque des revendications 1 à 6, **caractérisé en ce qu'**aucun autre gaz contenant de l'hydrogène moléculaire, en dehors du gaz de circulation, n'est introduit dans la zone de réaction.

8. Procédé selon l'une quelconque des revendications 1 à 7, **caractérisé en ce que** le gaz de réaction introduit dans la zone de réaction contient de la vapeur d'eau.

9. Procédé selon l'une quelconque des revendications 1 à 8, **caractérisé en ce que** la quantité partielle du gaz produit prélevé de la zone de réaction, recyclée comme gaz de circulation dans la zone de réaction, représente 20 à 80% de la quantité de gaz produit prélevée.

10. Procédé selon l'une quelconque des revendications 1 à 9, **caractérisé en ce que** la quantité partielle du gaz produit, non recyclée comme gaz de circulation dans la zone de réaction, est utilisée, le cas échéant après une séparation partielle des composants qui sont contenus, différents dudit au moins un hydrocarbure déshydrogéné, à des fins d'oxydation partielle et/ou d'ammoxydation partielle de l'hydrocarbure déshydrogéné.

11. Procédé selon l'une quelconque des revendications 1 à 10, **caractérisé en ce que** le gaz de réaction contient, sous forme d'ajout, un deuxième gaz de circulation provenant d'une oxydation partielle et/ou d'une ammoxydation partielle de l'hydrocarbure déshydrogéné, contenant ledit au moins un hydrocarbure à déshydrogéner.

12. Procédé selon l'une quelconque des revendications 1 à 11, **caractérisé en ce que** le deuxième gaz de circulation, recyclé à partir de l'oxydation partielle et/ou de l'ammoxydation partielle de l'hydrocarbure déshydrogéné dans la zone de réaction est le seul gaz contenant de l'oxygène moléculaire qui est introduit dans la zone de réaction.

13. Procédé selon l'une quelconque des revendications 1 à 12, **caractérisé en ce qu'**il est réalisé dans un réacteur à circulation à éjecteur à jet.

14. Réacteur, contenant au moins un garnissage de catalyseur, qui convient pour la déshydrogénation catalytique d'hydrocarbures à déshydrogéner, et au moins un éjecteur à jet, le garnissage catalytique étant conçu comme lit fixe catalytique et ledit au moins un éjecteur à jet comprenant une tuyère, un tube de mélange, un diffuseur et une tubulure d'aspiration.
